# EUROPEAN PATENT SPECIFICATION

(11) **EP 4 052 763 B1**
(45) Date of publication and mention of the grant of the patent: **24.12.2025**
(21) Application number: 22159446.8
(22) Date of filing: 01.03.2022
(51) Int. Cl.: A61Q 19/00, A61K 8/37, A61K 8/92, A61P 17/02, A61P 17/06, A61P 17/08, A61P 17/10

(54) **FORMULATION FOR EXTERNAL APPLICATION AND ITS USE**
FORMULIERUNG ZUR ÄUSSEREN ANWENDUNG UND IHRE VERWENDUNG
FORMULATION POUR APPLICATION EXTERNE ET SON UTILISATION

(30) Priority: 01.03.2021 PL 43719121
(43) Date of publication of application: 07.09.2022
(73) Proprietor: Ester Labs OÜ, 10141, Estonia, Tallinn (EE)
(72) Inventor: BODNAR, Bartosz, 05-509 Józefoslaw (PL); WARZECHA, Katarzyna, 05-509 Józefoslaw (PL)
(74) Representative: Piotrowicz, Alicja

(56) References cited:
- EP-A1- 2 974 604
- EP-B1- 2 713 777
- RO-B1- 129 084
- RO-B1- 129 086
- US-A1- 2011 098 495
- US-A1- 2012 213 715
- US-A1- 2014 249 227
- US-A1- 2016 206 588
- US-A1- 2017 165 280
- US-A1- 2019 374 552

## Description

The invention relates to a formulation for external application containing alkyl esters of polyunsaturated fatty acids, and said formulation for use in a method for care and/or supporting treatment and/or treatment of skin prone to inflammations of various etiologies, requiring special care and regeneration due to the tendency for inflammation, including alleviating skin inflammation and/or combating skin problems associated with inflammation and/or enhancing skin regeneration.

Skin is called the largest organ in the human body. It performs many functions important for human life, including, among others, serving as a protective barrier, taking part in gas exchange, performing a thermoregulatory function and many others. Therefore, it is not surprising that keeping human skin in a healthy condition is of interest to many scientists and entrepreneurs. Skin conditions can affect various areas of the skin such as the epidermis, glands, hair follicles, or nails. They may also have various causes, specific locations, or may exhibit specific features of lesions.

An increasingly common skin disease in the global population is atopic dermatitis (AD), which is a chronic inflammation of the skin that affects both children and adults. The exact cause of the disease is unknown, and it is even speculated that the development of AD may be due to the overlapping of various genetic, environmental and immunological factors. Atopic dermatitis is a medical condition that significantly worsens the patient's quality of life, in particular, because the disease is characterized by periods of disappearance of the disease symptoms, and then recurrence of intensified symptoms of the disease, which include, for example, itching, skin lesions, often exudative, or dry skin. It should also be emphasized that atopic dermatitis can manifest itself on many areas of the human body, including the areas of limbs flexion, hands, torso and even on the face. Patients with atopic dermatitis are advised to implement a long-term treatment process, which is often complex and requires both pharmacological intervention and the patients to follow strict discipline of non-pharmacological procedures. And so, the most common methods of atopic dermatitis treatment include rebuilding of the skin lipid coat, following a diet, or avoiding significant temperature changes, which may intensify the symptoms of the condition. Patients with AD are also advised to avoid the use of many cosmetics, which may contain substances that could aggravate the symptoms of the disease. In turn, pharmacological treatment uses glucocorticosteroids in the area of skin lesions or calcineurin inhibitors, although in severe forms of the disease, patients are also treated with immunosuppressants. Currently, there are many solutions on the market, designed to prevent, treat or alleviate skin conditions, and these solutions use countless chemical compounds as active substances and come in various forms, for example, gel or oral tablets.

In addition to atopic dermatitis, more and more people suffer from skin diseases related to cyclical or occasional inflammations of various etiologies. Such conditions include folliculitis, seborrheic dermatitis, and acne in both teenagers and adults. The etiologies of skin diseases and conditions are very broad, often associated with environmental factors such as increased environmental pollution or food, which in industrial production contains many noxious compounds, antibiotics or hormones. In the treatment of this type of skin conditions, antibiotics, steroids and retinoids are used. Depending on the symptoms and their severity, therapy with creams, ointments or gels based on benzoyl peroxide or adapalene is sometimes used. The treatment is usually long-term and, in addition to external therapy, systemic treatment is also used.

Polyunsaturated fatty acids, also known as omega acids, are very popular in the field of supporting treatment, preventing skin diseases and skin care. It has been shown many times that omega-3 and omega-6 acids are necessary for the proper growth, development and functioning of various organs in the human body. It is speculated that they exhibit comprehensive multi-organ activity, for instance, unsaturated omega fatty acids can have a positive effect on the synthesis of hormones responsible for well-being and they can alleviate inflammation and allergies. Some of these pro-health effects have already been formed into approved health claims, and many other applications are still under approval or research by centres around the world. Consuming omega fatty acids has a positive effect on HDL levels, while lowering LDL levels, and allows to regulate the level of triglycerides. In addition, omega acids help regulate blood glucose levels and regulate the immune system. At the same time, it has been proven that omega acids can have a beneficial effect on the condition of the skin, hair and nails. Omega-3 fatty acids include those unsaturated fatty acids in which the last double bond in the carbon chain is located at the third carbon atom from the end of the hydrocarbon chain. The omega-3 fatty acids include, among others, eicosapentaenoic acid (EPA), docosahexaenoic acid (DHA) and α-linolenic acid (ALA). Omega-6 fatty acids include those unsaturated fatty acids in which the last double bond is located at the sixth carbon atom from the end of the hydrocarbon chain. The omega-6 acids include, among others, linoleic acid (LA), γ-linolenic acid (GLA) and arachidonic acid (ARA). Omega-9 fatty acids include those unsaturated fatty acids in which the last double bond is located at the ninth carbon atom from the end of the of the hydrocarbon chain. The omega-9 acids include, but are not limited to, oleic acid, erucic acid, and nervonic acid.

The source of the omega fatty acids can be, for example, oils such as linseed oil, rapeseed oil, soybean oil, olive oil, sunflower oil, corn oil, sesame oil, and grape seed oil; fats such as fatback, lard, and butter; and fish such as halibut, eel, herring, mackerel, salmon, flounder, hake, and sardine.

The prior art relates to compositions containing omega fatty acids or omega fatty acid esters, as well as their applications and methods of preparation.

The Polish patent application No. P.408509 discloses a method of producing a mixture of vegetable fatty acid ethyl esters with a high content of *cis* isomers of unsaturated fatty acids. Also, disclosed is a mixture of vegetable fatty acid ethyl esters with a content of *cis* isomers of omega fatty acids of at least 95% of the original *cis* isomer content of omega-3, omega-6 or omega-9 unsaturated fatty acids in the vegetable oil used to prepare the esters.

CN patent application No. 102994236 A discloses a method of obtaining fatty acid ethyl ester with an omega-3 content greater than 90 percent by using fish oil as a raw material. The obtained omega-3 fatty acid ethyl ester can be used as a dietary supplement, drug additive, food additive.

US Patent No. 5,472,705 discloses topical pharmaceutical compositions containing esters of polyunsaturated omega-3 acids, namely esters of eicosapentaenoic acid (EPA) and docosahexaenoic acid (DHA) derived from fish. Phenolic antioxidants and adjuvants have been added to such formulations to overcome the unpleasant odours that naturally occur when these active ingredients are oxidized. The presented formulations have proven to be useful in the treatment of lesions, in particular in the treatment of psoriasis, phlebitis and related pathologies.

International patent application WO 2013150384 A1 discloses a pharmaceutical composition for oral administration containing vitamin D and at least 75% of at least one fatty acid selected from eicosapentaenoic acid (EPA) and docosahexaenoic acid (DHA) in the form of an ethyl ester, derived from fish oils. The pharmaceutical composition can be used in the prevention and treatment of psoriasis, as a dietary supplement, food additive, OTC supplement, medical food or pharmaceutical grade supplement.

European patent application EP 2974604 A1 discloses a process for producing a mixture of ethyl esters of vegetable fatty acids with a high content of cis-unsaturated fatty acid isomers of omega-3, omega-6 and omega-9 comprising obtaining a mixture of vegetable oils, trans-esterifying this feedstock with ethanol, evaporating and separating the ester phase and purifying. The mixture comprises at least 95% omega cis fatty acid of the initial cis isomer content of the unsaturated fatty acids omega-3, omega-6 and omega-9 in a vegetable oil. As vegetable oils, evening primrose seed oil and linseed oil is used, both oils containing a mixture of omega-3, omega-6 and omega-9 polyunsaturated fatty acids. The resulting oil mixture of the ethyl esters can be a product on its own, or else can be further processed (mixed with additional substances) and then apportioned.

Romanian patent RO 129 086 B1 and Romanian patent RO 129 084 B1 disclose a process for the preparation of ethyl esters of omega-3, omega-6 and omega-9 polyunsaturated fatty acids either from refined ostrich oil or from refined bear oil. Both oils contain a mixture of omega-3, omega-6 and omega-9 polyunsaturated fatty acids, and as such, by esterifying the fatty acids contained in the oil, a mixture of the three esters is obtained.

Further, a US patent application US 2014/249227 A1 discloses the use of a lipid composition comprising a daily dosage of linoleic acid (LA), alpha-linolenic acid (ALA), gamma-linolenic acid (GLA) and stearidonic acid (SDA), for use in a method of alleviating or treating skin problems selected from dryness of skin, tightness of skin, irritation of skin, local reddening of skin, itching of skin, thickening of skin, exfoliation of skin, atopic dermatitis, psoriasis, eczema and other skin problems associated with impaired skin barrier function. The lipid composition may comprise free omega-3, omega-6 and omega-9 fatty acids, or derivatives of free fatty acids such as fatty acid ethyl esters. The omega-3, omega-6 and omega-9 fatty acids are obtained from natural oils selected from safflower, soybean, corn, sunflower, grape seed, poppy seed, hempseed, wheat germ, cottonseed, walnut, evening primrose oil, echium oil and linseed oil. The lipid composition is in the form of dry powders, granules, pills, tablets, capsules, lozenges, dry products for reconstitution with water or other suitable carrier, aqueous or oily solutions or suspensions, gels, pastes, emulsions or syrups and is administered orally in the form of a dietary supplement, a nutritional supplement and a supplementary food.

It is important that the currently used compositions of omega polyunsaturated fatty acid esters are based on omega-3 acids from marine organisms, mainly on docosahexaenoic acid (DHA) and eicosapentaenoic acid (EPA). DHA and EPA are organic chemical compounds from the group of omega-3 polyunsaturated fatty acids. EPA acid consists of 20 carbon atoms and 5 double bonds. DHA, as the last and the longest omega-3 acid, has 22 carbon atoms and 6 double bonds. They are members of the essential fatty acids (EFAs). Their source includes algae and fish that feed on algae.

However, the use of compositions containing omega-3, 6 and 9 fatty acid alkyl esters based on omega-3 acids derived from marine organisms (EPA/DHA), which are present in high levels in fish oils, is currently a problem due to the fact of various inconveniences. The extraction of EFAs from fish is not sustainable, and the concentration of DHA and EPA in fish oil varies significantly depending on the location, season and phytoplankton availability. Moreover, the available amounts of fish oils are insufficient due to overfishing and environmental threats, so the growing need for EFAs cannot be met with the use of fish oil without the risk of serious damages or even annihilation of the population of marine organism. Currently, already 60 to 70% of the world's fish oil production comes from Peru and Chile, where the already decimated population of anchovies is being exterminated on a gigantic scale. Moreover, the pollution of the seas and oceans made it practically impossible to obtain the raw material uncontaminated with heavy metals, microplastics or industrial pollutants, which decreased the quality of active ingredients.

Moreover, the vast majority of the state of the art solutions relate to omega acid esters compositions which are intended for oral use as dietary supplements, food additives. Systemic use is widespread, but it is associated with insufficient effectiveness of these formulations, as well as the risk of undesirable side effects.

An additional difficulty in developing an effective formulation for application to the skin is overcoming the unpleasant odour accompanying the oxidation of DHA and EPA derived from marine organisms during their transport and storage.

Thus, despite many solutions used in the prior art, there is still a need for a formulation intended for application to the skin which, on the one hand, will be effective in alleviating, i.e. nourishing, treating or healing inflammatory conditions of the skin of various etiologies, and on the other hand, will eliminate the concerns related to the use of active ingredients of marine origin.

The present inventors were faced with the abovementioned need and disadvantages of the prior art, and were the first to develop a formulation for external use, i.e. directly to the skin, containing ethyl esters of omega-3, 6 and 9 polyunsaturated fatty acids exclusively of plant origin, in a sufficiently high concentration. The obtained formulation containing, as an active ingredient, only plant-derived omega-3, 6 and 9 fatty acid esters, in high concentration, showed an unexpectedly high anti-inflammatory and regenerative effect, and turned out to be exceptionally effective in alleviating inflammations of various etiologies, such as inflammations in atopic dermatitis (AD), seborrheic dermatitis or folliculitis. For the first time, a formulation for direct application to the skin was developed that contains plant-derived omega-3, 6 and 9 fatty acid esters. At the same time, the formulation developed by the inventors overcame the problems related to the threat to the population of marine organisms, the contamination of the raw material as well as the oxidation of active ingredients of marine origin. Obtaining the raw material for the production of the formulation based on ethyl esters of omega-3, 6 and 9 polyunsaturated fatty acids of plant origin is definitely more environmentally friendly and can be implemented in a sustainable manner without any environmental damage.

The objective of the invention is to provide a formulation for external use containing alkyl esters of polyunsaturated fatty acids, characterized in that it contains ethyl esters of omega-3, omega-6 and omega-9 polyunsaturated fatty acids exclusively of plant origin, in a concentration of 8% to 45% with respect to the total weight of the formulation.

The high concentration of plant-derived ethyl esters of omega-3, omega-6 and omega-9 polyunsaturated fatty acid in the formulation according to the invention provides an unexpectedly high formulation effectiveness.

The formulation according to the invention containing omega-3, 6 and 9 fatty acid ethyl esters exclusively of plant origin contains, among others, α-linolenic acid ethyl ester (omega-3), γ-linolenic acid ethyl ester (omega-6), linoleic acid ethyl ester (omega-6), oleic acid ethyl ester (omega-9), obtained from vegetable oils from oil plants.

According to the invention, the source of the omega-3, omega-6 and omega-9 polyunsaturated fatty acid ethyl esters is vegetable oil selected from the group comprising: linseed oil *(Linum* L.), borage oil (*Borago* L.), black cumin oil (*Nigella* L.), pumpkin seed oil (*Cucurbita* L.), walnut oil (*Juglans* L.) and chia seed oil (*Salvia L.).*

The main active ingredient of the formulation according to the invention, found in the highest concentration in vegetable oils, is α-linolenic acid (omega-3, ALA). Unlike EPA and DHA, plant acids derived from terrestrial plants are shorter-chain acids. Omega-3 ALA contains only 18 carbon atoms and has 3 double bonds. These acids were for a long time neglected in research, because it was believed that their effect on the body is very limited and only EPA and DHA exhibit pro-health effects. Meanwhile, plant-derived polyunsaturated fatty acids such as ALA are essential for the proper growth and development, they regulate cholesterol levels, and have anti-inflammatory effects, both systemically and externally.

Fatty acids and omega-3, 6, 9 acid esters are necessary to rebuild the proper hydro-lipid coat of the skin. Omega 3 and 6 acids (a-linolenic acid, linoleic acid, γ-linolenic acid) improve skin hydration, reduce the feeling of roughness and tightness, reduce the predisposition to adverse changes of the epidermis. In addition, omega 3 and 6 acids are absorbed by the stratum corneum and build into the intercellular cement, reducing water loss through the epidermis. They improve skin elasticity and restore its healthy appearance. In turn, omega-9 acids (oleic acid) have moisturizing and softening effect on the epidermis.

Preferably, the source of the ethyl esters of omega-3, omega-6 and omega-9 polyunsaturated fatty acid in the formulation according to the invention is linseed oil (*Linum* L.). In such an embodiment, the formulation contains α-linolenic acid ethyl ester (omega-3), linoleic acid ethyl ester (omega-6), oleic acid ethyl ester (omega-9), palmitic acid ethyl ester, and stearic acid ethyl ester in the ratio, respectively, 57% : 16% : 15% : 7% : 5%.

Preferably, the formulation according to the invention additionally contains at least one plant-based cosmetic oil. Most preferably, the formulation of the invention contains:
a) ethyl esters of omega-3, omega-6 and omega-9 polyunsaturated fatty acids derived from linseed oil (*Linum* L.), in a concentration of 8 to 45 wt.%; and
b) camelina oil (*Camelina* L.), in a concentration of 0.05 to 5 wt.%; and/or
c) evening primrose oil (*Oenothera* L.), in a concentration of 0.05 to 3 wt.%; and/or
d) calendula oil extract (*Calendula* L.), in a concentration of 0.01 to 4 wt.%.
   wherein wt.% defines the concentration of a given ingredient with respect to the total weight of the formulation.

Ethyl esters of omega-3, omega-6 and omega-9 polyunsaturated fatty acids of plant origin in combination with three vegetable oils: camelina, evening primrose and calendula oils additionally support the process of the epidermis regeneration and enhance anti-inflammatory effects. The combination of these ingredients, in the adequate proportions, resulted in a formulation with unexpectedly high effectiveness, which has an exceptionally high anti-inflammatory and regenerative effect.

Preferably, the formulation according to the invention further contains at least one additional active ingredient selected from the group comprising: D-panthenol, propolis, collagen, aloe extract, ozone, and copper.

The formulation according to the invention may be a cosmetic formulation or a pharmaceutical formulation.

The formulation according to the invention further contains cosmetically or pharmaceutically acceptable additives, such as moisturizers, stabilizers, preservatives, emulsifiers, emollients, antioxidants, fragrances, or solvents.

Moisturizers that can be used in the preparation of the formulation of the invention include, for example, maltooligosyl glucoside, hydrogenated starch hydrolysate; glycerine; 1,3-propanediol.

Stabilizers that can be used in the preparation of the formulation of the invention include, for example, magnesium sulphate.

Preservatives that can be used in the preparation of the formulation of the invention include, for example: dehydroacetic acid, benzoic acid, phenoxyethanol; benzyl alcohol, salicylic acid, glycerine, sorbic acid; phenoxyethanol, ethylhexylglycerin; water, benzyl alcohol, potassium sorbate, sodium benzoate.

Emulsifiers that can be used in the preparation of the formulation of the invention include, for example, polyethylene glycol dipolyhydroxystearate; isostearic acid and polyglycerin-3 diester; cetylstearyl oleate sorbitol oleate; glycerol stearate.

Emollients that can be used in the preparation of the formulation of the invention include, for example, paraffin oil; petroleum jelly; dioctyl acid ether; camelina oil; evening primrose oil; borage oil; coconut oil; sweet almond oil, triglycerides of caprylic and capric acid; isostearyl isostearate; or shea butter.

Antioxidants that can be used in the preparation of the formulation of the invention include, for example, vitamin E acetate.

Fragrances that can be used in the preparation of the formulation of the invention include, for example, essential oils; natural or synthetic fragrance compositions.

Solvents that can be used in the preparation of the formulation of the invention include, for example, demineralized water.

The formulation according to the invention is intended to be applied directly to the skin and is in the form of a cream, oil, gel, or emulsion. Preferably, the formulation according to the invention is in the form of a water-in-oil emulsion.

The invention further relates to the formulation according to the invention for use for care and/or supporting treatment and/or treatment of skin prone to inflammations of various etiologies, including alleviating skin inflammation and/or combating skin problems associated with inflammation and/or enhancing skin regeneration.

Advantageously, according to the invention, the formulation of the invention can be applied to skin prone to inflammations of various etiologies, wherein such skin can be atopic skin, sensitive skin, combination skin, couperose skin, dry skin, problematic skin, skin prone to irritation and the epidermis damage.

The formulation of the invention can be used to alleviate inflammatory skin conditions, wherein the inflammatory skin conditions includes atopic dermatitis, seborrheic dermatitis, contact dermatitis, folliculitis, acne, psoriasis, skin irritation, and skin problems.

The subject of the invention is also the use of omega-3, omega-6 and omega-9 fatty polyunsaturated acid ethyl esters of plant origin in the preparation of a formulation for external use for care and/or supporting treatment and/or treatment of skin prone to inflammations of various etiologies, including alleviating skin inflammation and/or combating skin problems associated with inflammation and/or enhancing skin regeneration, wherein omega-3, omega-6 and omega-9 polyunsaturated fatty acid ethyl esters of plant origin for the preparation of the formulation according to the invention, high concentration of esters, ranging from 8 to 45% with respect to the total weight of the formulation, is crucial, which ensures unexpectedly high effectiveness of the formulation according to the invention.

The effectiveness of the formulation according to the invention has been tested and documented in the following examples and also in the figures, wherein:
Fig. 1 shows the effect of the formulation according to the invention on cracking, non-healing abdominal skin of an elderly and bedridden person.
Fig. 2 shows the effect of the formulation according to the invention on lichen and cheek skin lesions associated with severe food and inhalation allergies in a 5-year-old girl.
Fig. 3 shows the effect of the formulation according to the invention on atopic skin lesions of the ear, i.e. inflammation and scaly skin in a 9-year-old boy.
Fig. 4 shows the effect of the formulation according to the invention on cracking and ulcerated skin of the fingers of a middle-aged woman.
Fig. 5 shows the effect of the formulation according to the invention on atopic dermatitis, manifested by painful, bleeding and itchy eczema in a middle-aged woman.
Fig. 6 shows the effect of the formulation according to the invention on acute folliculitis in a 37-year-old man.
Fig. 7 shows the effect of the formulation according to the invention on skin inflammation associated with food allergy in an infant.
Fig. 8 shows the effect of the formulation according to the invention on atopic dermatitis in a one and a half year old boy

The invention is illustrated by the following examples. The following examples are intended to better understand the invention and do not limit its scope.

### Examples

### Example 1 - Active ingredient of the formulation according to the invention: pure omega 3, 6 and 9 polyunsaturated fatty acid ethyl esters

The active ingredient of the formulation according to the invention are pure omega 3, 6 and 9 polyunsaturated fatty acid ethyl esters, exclusively of plant origin.

The raw material used as an active ingredient of the formulation of the invention is a composition of omega 3, 6 and 9 polyunsaturated fatty acid ethyl esters obtained from plant oils. The source of these omega 3, 6 and 9 fatty acids was linseed oil (*Linum* L.). The physical and chemical properties of the raw material were examined and summarized in Table 1 below.

**Table 1: Physicochemical properties of omega 3, 6 and 9 polyunsaturated fatty acid ethyl esters**

| **Raw material name and chemical name** | **Physicochemical properties** | |
|---|---|---|
| Omega Esters | State of matter | Liquid |
| | Colour | Yellow |
| | Odour | Specific |
| | Peroxide value | < 1 meq O₂ |
| Ethyl esters of linolenic acid, linoleic acid and oleic acid | Acid value | < 4 mg KOH/g |
| | Density | 0.93 g/cm³ |
| | Heavy metals (As, Pb, Cd, Hg) [mg/kg] | Below the sensitivity of the method |
| | Chlorinated and organophosphorus pesticides, pyrethroids | Not present |
| | Ingredients [% m/V]: | |
| | α-Linolenic acid ethyl ester (n-3, ALA, Omega-3) | 57 |
| | Linoleic acid ethyl ester (n-6, LA, Omega-6) | 16 |
| | Oleic acid ethyl ester (n-9, Omega-9) | 15 |
| | Palmitic acid ethyl ester | 7 |
| | Stearic acid ethyl ester | 5 |
| | Stability | Stable under normal conditions |

The ester composition used as an active ingredient in the formulation according to the invention contains α-linolenic acid ethyl ester (n-3, ALA, Omega-3), linoleic acid ethyl ester (n-6, LA, Omega-6), oleic acid ethyl ester (n-9, Omega-9), palmitic acid ethyl ester and stearic acid ethyl ester, in the ratio, respectively, of 57% : 16% : 15% : 7% : 5%.

### Example 2 - Formulation of the invention containing omega-3, omega-6 and omega-9 polyunsaturated fatty acid ethyl esters in a concentration of 12%

The main active ingredient of the formulation according to the invention was the raw material described in Example 1, *i.e.* the composition of omega 3, 6 and 9 polyunsaturated fatty acid ethyl esters derived from linseed oil (*Linum* L.) in a concentration of 12%, based on the total weight of the formulation.

The qualitative and quantitative composition of the formulation according to the invention containing omega-3, omega-6 and omega-9 polyunsaturated fatty acid ethyl esters from linseed oil is presented in Table 2 below.

**Table 2: Qualitative and quantitative composition of the formulation according to the invention containing omega-3, omega-6 and omega-9 polyunsaturated fatty acid ethyl esters from linseed oil in a concentration of 12%**

| | **Raw material (commercial name)** | **INCI composition** | **Chemical name** | **%** | **Function** |
|---|---|---|---|---|---|
| **PHASE A** | | | | | |
| 1. | Demineralized Water | Aqua | Demineralized water | 62.0 | Solvent |
| 2. | MG-60 | Maltooligosyl Glucoside, Hydrogenated Starch Hydrolysate | Maltooligosyl glucoside, hydrogenated starch hydrolysate | 2.50 | Moisturizing ingredient |
| 3. | Magnesium Sulphate | Magnesium Sulphate | Magnesium sulphate | 0.70 | Stabilizing agent |
| 4. | Euxyl K703 | Dehydroacetic Acid, Benzoic Acid, Phenoxyethanol | Dehydroacetic acid, benzoic acid, phenoxyethanol | 1.00 | Preservative |

| **PHASE B** | | | | | |
|---|---|---|---|---|---|
| 5. | Cithrol DPHS | PEG-30 Dipolyhydroxystearate | Polyethylene glycol dipolyhydroxystearate | 2.00 | Emulsifier |
| 6. | Cithrol PG32IS | Polyglyceryl-3 Diisostearate | Diester of isostearic acid and polyglycerin-3 | 3.00 | Emulsifier |
| 7. | Vara 200 | Paraffinum Liquidum | Paraffin oil | 3.00 | Emollient |
| 8. | Paracera M | Microcrystalline Wax | Microcrystalline wax | 0.50 | Stabilizing/ thickening agent |
| 9. | Petroleum Jelly | Petrolatum | Petroleum jelly | 0.50 | Emollient |
| 10. | Hydrogenated Castor Oil | Hydrogenated Castor Oil | Hydrogenated castor oil | 0.50 | Stabilizing/ thickening agent |
| 11. | Cetiol OE | Dicaprylyl Ether | Dioctyl acid ether | 2.00 | Emollient |
| 12. | Vitamin E | Tocopheryl Acetate | Vitamin E acetate | 0.20 | Antioxidant |

| **PHASE C** | | | | | |
|---|---|---|---|---|---|
| 13. | Camelina Oil (from false flax) | Camelina Sativa Seed Oil | Camelina oil (from false flax) | 5.00 | Emollient |
| 14. | Evening Primrose Oil | Oenothera Biennis Seed Oil | Evening primrose oil | 3.00 | Emollient |
| 15. | Omega Esters | Linolenic Acid, Linoleic Acid, Oleic Acid ethyl esters | Linolenic acid, linoleic acid, oleic acid ethyl esters | 12.00 | Active ingredient |

| **PHASE D** | | | | | |
|---|---|---|---|---|---|
| 16. | D-Panthenol 75% | Panthenol | Panthenol | 1.00 | Active ingredient |
| 17. | Propolis | Propolis Cera, Alcohol Denat. | Ethanol propolis extract | 0.10 | Active ingredient |
| 18. | Calendula Officinalis Extract | Calendula Officinalis Flower Extract, Helianthus Annuus Seed Oil | Oil Calendula Officinalis extract (sunflower oil as extractant) | 1.00 | Active ingredient |

The formulation of the invention was prepared according to the procedure described below in Example 7.

### Example 3 - Formulation of the invention containing omega-3, omega-6 and omega-9 polyunsaturated fatty acid ethyl esters in a concentration of 8%

The main active ingredient of the formulation according to the invention was the raw material obtained in Example 1, *i.e.* the composition of omega 3, 6 and 9 polyunsaturated fatty acid ethyl esters derived from linseed oil (*Linum* L.) in a concentration of 8%, based on the total weight of the formulation.

The qualitative and quantitative composition of the formulation according to the invention containing omega-3, omega-6 and omega-9 polyunsaturated fatty acid ethyl esters from linseed oil is presented in Table 3 below.

**Table 3: Qualitative and quantitative composition of the formulation according to the invention containing omega-3, omega-6 and omega-9 polyunsaturated fatty acid ethyl esters from linseed oil in a concentration of 8%**

| | **Raw material (commercial name)** | **INCI composition** | **Chemical name** | **%** | **Function** |
|---|---|---|---|---|---|
| **PHASE A** | | | | | |
| 1. | Demineralized Water | Aqua | Demineralized water | 61.20 | Solvent |
| 2. | MG-60 | Maltooligosyl Glucoside, Hydrogenated Starch Hydrolysate | Maltooligosyl glucoside, hydrogenated starch hydrolysate | 2.50 | Moisturizing ingredient |
| 3. | Magnesium Sulphate | Magnesium Sulphate | Magnesium sulphate | 0.70 | Stabilizing agent |
| 4. | Euxyl K703 | Dehydroacetic Acid, Benzoic Acid, Phenoxyethanol | Dehydroacetic acid, benzoic acid, phenoxyethanol | 1.00 | Preservative |

| **PHASE B** | | | | | |
|---|---|---|---|---|---|
| 5. | Cithrol DPHS | PEG-30 Dipolyhydroxystearate | Polyethylene glycol dipolyhydroxystearate | 2.00 | Emulsifier |
| 6. | Cithrol PG32IS | Polyglyceryl-3 Diisostearate | Diester of isostearic acid and polyglycerin-3 | 3.00 | Emulsifier |
| 7. | Radia 7104 | Caprylic/Capric Triglyceride | Triglycerides of caprylic and capric acid | 3.00 | Emollient |
| 8. | Paracera M | Microcrystalline Wax | Microcrystalline wax | 0.50 | Stabilizing/ thickening agent |
| 9. | Shea Butter | Butyrospermum Parkii Butter | Shea butter | 1.00 | Emollient |
| 10. | Hydrogenate d Castor Oil | Hydrogenated Castor Oil | Hydrogenated castor oil | 0.50 | Stabilizing/ thickening agent |
| 11. | Cetiol OE | Dicaprylyl Ether | Dioctyl acid ether | 5.00 | Emollient |
| 12. | Vitamin E | Tocopheryl Acetate | Vitamin E acetate | 0.50 | Antioxidant |

| **PHASE C** | | | | | |
|---|---|---|---|---|---|
| 13. | Camelina Oil (from false flax) | Camelina Sativa Seed Oil | Camelina oil (from false flax) | 5.00 | Emollient |
| 14. | Evening Primrose Oil | Oenothera Biennis Seed Oil | Evening primrose oil | 3.00 | Emollient |
| 15. | Omega Esters | Linolenic Acid, Linoleic Acid, Oleic Acid ethyl esters | Linolenic acid, linoleic acid, oleic acid ethyl esters | 8.00 | Active ingredient |

| **PHASE D** | | | | | |
|---|---|---|---|---|---|
| 16. | D-Panthenol 75% | Panthenol | Panthenol | 1.00 | Active ingredient |
| 17. | Propolis | Propolis Cera, Alcohol Denat. | Ethanol propolis extract | 0.10 | Active ingredient |
| 18. | Calendula Officinalis Extract | Calendula Officinalis Flower Extract, Helianthus Annuus Seed Oil | Oil Calendula Officinalis extract (sunflower oil as extractant) | 2.00 | Active ingredient |

The formulation of the invention was prepared according to the procedure described below in Example 7.

### Example 4 - Formulation of the invention containing omega-3, omega-6 and omega-9 polyunsaturated fatty acid ethyl esters in a concentration of 20%

The main active ingredient of the formulation according to the invention was the raw material obtained in Example 1, *i.e.* the composition of omega 3, 6 and 9 polyunsaturated fatty acid ethyl esters derived from linseed oil (*Linum* L.) in a concentration of 20%, based on the total weight of the formulation.

The qualitative and quantitative composition of the formulation according to the invention containing omega-3, omega-6 and omega-9 polyunsaturated fatty acid ethyl esters from linseed oil is shown in Table 4 below.

**Table 4: Qualitative and quantitative composition of the formulation according to the invention containing omega-3, omega-6 and omega-9 polyunsaturated fatty acid ethyl esters from linseed oil in a concentration of 20%**

| | **Raw material (commercial name)** | **INCI composition** | **Chemical name** | **%** | **Function** |
|---|---|---|---|---|---|
| **PHASE A** | | | | | |
| 1. | Demineralized Water | Aqua | Demineralized water | 53.00 | Solvent |
| 2. | Glycerine | Glycerine | Glycerol | 3.00 | Moisturizing ingredient |

| **PHASE B** | | | | | |
|---|---|---|---|---|---|
| 3. | Olivem 1000 | Cetearyl Olivate, Sorbitan Olivate | Sorbitan esters and cetylstearyl esters of fatty acids from olive oil | 4.00 | Emulsifier |
| 4. | Lasemul 92AE | Glyceryl Stearate SE | Glyceryl stearate | 2.00 | Co-emulsifier |
| 5. | Vegarol 1618 | Cetearyl Alcohol | Cetylstearyl alcohol | 2.00 | Thickening agent |
| 6. | Cetyl Alcohol | Cetyl Alcohol | Cetyl alcohol | 1.00 | Thickening agent |
| 7. | Shea Butter | Butyrospermum Parkii Butter | Shea butter | 0.50 | Emollient |
| 8. | Coconut Oil | Cocos Nucifera Oil | Coconut oil | 0.50 | Emollient |
| 9. | Radia 7104 | Caprylic/Capric Triglyceride | Triglycerides of caprylic and capric acid | 3.00 | Emollient |
| 10. | Crodamol ISIS | Isostearyl Isostearate | Isostearyl isostearate | 1.00 | Emollient |
| 11. | Cetiol OE | Dicaprylyl Ether | Dioctyl acid ether | 2.00 | Emollient |
| 12. | Vitamin E | Tocopheryl Acetate | Vitamin E acetate | 0.50 | Antioxidant |

| **PHASE C** | | | | | |
|---|---|---|---|---|---|
| 13. | Camelina Oil (from false flax) | Camelina Sativa Seed Oil | Camelina oil (from false flax) | 2.00 | Emollient |
| 14. | Evening Primrose Oil | Oenothera Biennis Seed Oil | Evening primrose oil | 1.00 | Emollient |
| 15. | Omega Esters | Linolenic Acid, Linoleic Acid, Oleic Acid ethyl esters | Linolenic acid, linoleic acid, oleic acid ethyl esters | 20.00 | Active ingredient |
| 16. | Borage Oil | Borago Officinalis Seed Oil | Borage seed oil | 2.00 | Emollient |

| **PHASE D** | | | | | |
|---|---|---|---|---|---|
| 17. | D-Panthenol 75% | Panthenol | Panthenol | 1.00 | Active ingredient |
| 18. | Geogard ECT | Benzyl Alcohol, Salicylic Acid, Glycerine, Sorbic Acid | Benzyl alcohol, salicylic acid, glycerol, sorbic acid | 1.00 | Preservative |
| 19. | Fragrance Composition | Parfum | Fragrance composition | 0.50 | Fragrance |

The formulation of the invention was prepared according to the procedure described below in Example 7.

### Example 5 - Formulation of the invention containing omega-3, omega-6 and omega-9 polyunsaturated fatty acid ethyl esters in a concentration of 30%

The main active ingredient of the formulation according to the invention was the raw material obtained in Example 1, *i.e.* the composition of omega 3, 6 and 9 polyunsaturated fatty acid ethyl esters derived from linseed oil (*Linum* L.) in a concentration of 30%, based on the total weight of the formulation.

The qualitative and quantitative composition of the formulation according to the invention containing omega-3, omega-6 and omega-9 polyunsaturated fatty acid ethyl esters from linseed oil is shown in Table 5 below.

**Table 5: Qualitative and quantitative composition of the formulation according to the invention containing omega-3, omega-6 and omega-9 polyunsaturated fatty acid ethyl esters from linseed oil in a concentration of 30%**

| | **Raw material (commercial name)** | **INCI composition** | **Chemical name** | **%** | **Function** |
|---|---|---|---|---|---|
| **PHASE A** | | | | | |
| 1. | Demineralized Water | Aqua | Demineralized water | 52.40 | Solvent |
| 2. | Zemea | Propanediol | 1,3-propanediol | 3.00 | Moisturizing ingredient |

| **PHASE B** | | | | | |
|---|---|---|---|---|---|
| 3. | Cosmowax-D | Cetearyl Alcohol Ceteareth-20 | A mixture of cetostearyl alcohol and ethoxylated (20 EO) cetostearyl alcohol | 3.00 | Emulsifier |
| 4. | Lasemul 92AE | Glyceryl Stearate SE | Glyceryl stearate | 2.00 | Emulsifier |
| 5. | Cosmedia SP | Sodium Polyacrylate | Sodium polyacrylate | 0.60 | Emulsion stabilizing agent |
| 6. | Shea Butter | Butyrospermum Parkii Butter | Shea butter | 1.00 | Emollient |
| 7. | Cetiol OE | Dicaprylyl Ether | Dioctyl acid ether | 2.00 | Emollient |
| 8. | Vitamin E | Tocopheryl Acetate | Vitamin E acetate | 0.50 | Antioxidant |

| **PHASE C** | | | | | |
|---|---|---|---|---|---|
| 9. | Camelina oil (from false flax) | Camelina Sativa Seed Oil | Camelina oil (from false flax) | 2.00 | Emollient |
| 10. | Evening primrose oil | Oenothera Biennis Seed Oil | Evening primrose oil | 1.00 | Emollient |
| 11. | Omega Esters | Linolenic Acid, Linoleic Acid, Oleic Acid ethyl esters | Linolenic acid, linoleic acid, oleic acid ethyl esters | 30.00 | Active ingredient |

| **PHASE D** | | | | | |
|---|---|---|---|---|---|
| 12. | D-Panthenol 75% | Panthenol | Panthenol | 1.00 | Active ingredient |
| 13. | Optiphen BSB-W | Aqua, Benzyl Alcohol, Potassium Sorbate, Sodium Benzoate | Water, benzyl alcohol, potassium sorbate, sodium benzoate | 1.00 | Preservative substance |
| 14. | Fragrance Composition | Parfum | Fragrance composition | 0.50 | Fragrance |

The formulation of the invention was prepared according to the procedure described below in Example 7.

### Example 6 - Formulation of the invention containing omega-3, omega-6 and omega-9 polyunsaturated fatty acid ethyl esters in a concentration of 45%

The main active ingredient of the formulation according to the invention was the raw material obtained in Example 1, *i.e.* the composition of omega 3, 6 and 9 polyunsaturated fatty acid ethyl esters derived from linseed oil (*Linum* L.) in a concentration of 45%, based on the total weight of the formulation.

The qualitative and quantitative composition of the formulation according to the invention containing omega-3, omega-6 and omega-9 polyunsaturated fatty acid ethyl esters from linseed oil is shown below in Table 6.

**Table 6: Qualitative and quantitative composition of the formulation according to the invention containing omega-3, omega-6 and omega-9 polyunsaturated fatty acid ethyl esters from linseed oil in a concentration of 45%**

| | **Raw material (commercial name)** | **INCI composition** | **Chemical name** | **%** | **Function** |
|---|---|---|---|---|---|
| **PHASE A** | | | | | |
| 1. | Demineralized Water | Aqua | Demineralized water | 42.70 | Solvent |
| 2. | Zemea | Propanediol | 1,3-propanediol | 2.00 | Moisturizing ingredient |
| 3. | Magnesium Sulphate | Magnesium Sulphate | Magnesium sulphate | 0.70 | Stabilizing agent |
| 4. | Euxyl PE9010 | Phenoxyethanol, Ethylhexylglycerin | Phenoxyethanol, Ethylhexylglycerol | 1.00 | Preservative substance |
| **PHASE B** | | | | | |
| 5. | Cithrol DPHS | PEG-30 Dipolyhydroxystearate | Polyethylene glycol dipolyhydroxystearate | 2.00 | Emulsifier |
| 6. | Cithrol PG32IS | Polyglyceryl-3 Diisostearate | Diester of isostearic acid and polyglycerin-3 | 3.00 | Emulsifier |
| 7. | Sweet Almond Oil | Prunus Amygdalus Dulcis Oil | Sweet almond oil | 0.50 | Emollient |
| 8. | Paracera M | Microcrystalline Wax | Microcrystalline wax | 0.50 | Stabilizing/ thickening agent |
| 9. | Coconut Oil | Cocos Nucifera Oil | Coconut oil | 0.50 | Emollient |
| 10. | Hydrogenated Castor Oil | Hydrogenated Castor Oil | Hydrogenated castor oil | 0.50 | Stabilizing/ thickening agent |
| 11. | Cetiol OE | Dicaprylyl Ether | Dioctyl acid ether | 0.50 | Emollient |
| 12. | Vitamin E | Tocopheryl Acetate | Vitamin E acetate | 0.50 | Antioxidant |
| PHASE C | | | | | |
| 13. | Camelina oil (from false flax) | Camelina Sativa Seed Oil | Camelina oil (from false flax) | 0.05 | Emollient |
| 14. | Evening primrose oil | Oenothera Biennis Seed Oil | Evening primrose oil | 0.05 | Emollient |
| 15. | Omega Esters | Linolenic Acid, Linoleic Acid, Oleic Acid ethyl esters | Linolenic acid, linoleic acid, oleic acid ethyl esters | 45.00 | Active ingredient |

| **PHASE D** | | | | | |
|---|---|---|---|---|---|
| 16. | Fragrance Composition | Parfum | Fragrance composition | 0.50 | Fragrance |

The formulation of the invention was prepared according to the procedure described below in Example 7.

### Example 7 - Preparation of the formulation according to the invention containing omega-3, omega-6 and omega-9 polyunsaturated fatty acid ethyl esters

The formulation of the invention has been produced in accordance with the requirements of Good Manufacturing Practice (GMP). All raw materials indicated in Tables 2-6 and, respectively in Examples 2-6, grouped to raw materials of the PHASE A, B, C and D according to the order of their addition, were added in the concentrations given therein.

All PHASE B raw materials were placed in the main tank. Everything was heated to 75-80°C until all components were completely melted.

The PHASE A raw materials were placed in a separate tank and heated to a temperature of 75-80°C. Thereafter, it was stirred until the components were completely dissolved.

The phases were combined at a temperature of 75-80°C. For this purpose, the hot components of PHASE A were added to the main tank (PHASE B). The mixture was mixed and then homogenized (3500-4000 rpm) for about 10 minutes (depending on the size of the production batch). The vacuum was maintained. After homogenization was completed, the cooling process was started. The mixture was cooled to approx. 60°C while stirring constantly.

In a separate tank, the components of PHASE C were heated to a temperature of 30°C (not more).

The PHASE C components heated to 30°C were added to the main tank at 60°C. The mixture was mixed and then homogenized (10 minutes). The cooling process was initiated.

The PHASE D components were added to the main tank at 30-35°C. The mixture was mixed. After cooling the mixture to 25°C, it was homogenized for 10 minutes, until the desired consistency of the product was obtained, and then the production process was completed.

A formulation was obtained in the form of a water-in-oil emulsion containing ethyl esters of omega 3, 6 and 9 polyunsaturated fatty acids derived from linseed oil *(Linum* L.). Then, the properties of the resulting formulation, such as appearance, consistency, colour, odour, as well as thermal stability and microbiological purity, were examined. The results are summarized in Table 7.

**Table 7: Properties of the formulation according to the invention containing omega-3, omega-6 and omega-9 polyunsaturated fatty acid ethyl esters from linseed oil (Linum L.)**

| **No.** | **Properties** | **Requirements** | **Evaluation method** |
|---|---|---|---|
| 1. | Appearance | homogeneous, smooth emulsion, without mechanical impurities | organoleptic |
| 2. | Colour | cream - yellow, consistent with the standard | organoleptic |
| 3. | Odour | characteristic odour of the raw materials used, consistent with the standard | organoleptic |
| 4. | Thermal stability at temp. +40°C/1 month | stable | thermal |
| 5. | Thermal stability at temp. 5°C/1 month | stable | thermal |
| 6. | Thermal stability at temp. -5°C/1 month | stable | thermal |
| 7. | **Microbiological purity:** | | |
| | - the total number of mesophilic aerobic microorganisms | <1000 cfu/g | PN-EN ISO 21149:2009 |
| | *- Pseudomonas aeruginosa* | not present in 1 g sample | PN-EN ISO 22717:2010 |
| | *- Staphyllococcus aureus* | not present in 1 g sample | PN-EN ISO 22718:2010 |
| | *- Candida albicans* | not present in 1 g sample | PN-EN ISO 18416:2009 |
| | *- Escherichia coli* | not present in 1 g sample | PN-EN ISO 21150:2010 |

The formulation was stable under all temperature conditions. No separation of the emulsion or other changes except odour were observed. After each control period, an intensification of the specific odour of the active ingredients (omega acid esters, evening primrose oil and camelina oil) was observed. Due to the high content of natural vegetable oils and the active ingredient (omega esters), and the tendency of these substances to oxidize quickly, the shelf life of the formulation according to the invention is max. 12 months from the production date.

### Example 8 - Dermatological examination for possible contact allergy and irritation reaction after using the formulation according to the invention

In order to assess the occurrence of a possible contact allergy (delayed type allergy) and/or irritation reaction after using the formulation according to the invention, dermatological patch tests/contact tests were performed. Sensitization occurs as a result of contact with an antigen that penetrates the skin. The antigen is recognized by Langerhans cells and presented to T lymphocytes. Specifically sensitized lymphocytes multiply in the regional lymph node. After the re-penetration of the antigen, rapid proliferation of specifically sensitized lymphocytes occurs, which, by releasing pro-inflammatory substances (lymphokines), trigger an inflammatory process at the area of skin contact with the sensitizing substance. The lesions usually affect the upper skin layers (epidermis and dermal papillae) and are characterized by erythema, blistering and skin scaling.

The study was carried out in accordance with the research procedure of the Regulation of the European Parliament and of the Council (EC) No. 1223/2009 of November 30, 2009, on cosmetic products, and COLIPA Guidelines "Product test Guidelines for the Assessment of Human Skin Compatibility 1997", "Guidelines for the Evaluation of the Efficacy of Cosmetic Products 2008".

The formulation according to the invention in the specific form obtained in Example 2 was applied once to the skin by means of special containers fixed on a hypoallergenic adhesive. The containers were filled with the test formulation and then glued to the skin of the back in the scapular area. The test subjects were warned that the back should not be wet during the tests, and sudden movements and sweating should also be avoided. The patches with the test formulation were left on the skin of the test subjects for 48 hours. After the adhesives with the containers were removed, excess test formulation was removed by pressing a paper towel against the skin. The skin reaction was evaluated immediately after removing the adhesives, after 72 hours, after 96 hours and after 7 days according to the scale which is consistent with the scale generally accepted in dermatological tests.

The selection of the test subjects was carried out in accordance with the research procedure taking into account the Declaration of Helsinki of 1964 (with subsequent revisions) and the currently applicable Polish and European legal regulations, as well as the Cosmetics Europe guidelines using the inclusion and exclusion criteria.

15 women/men, aged 22 to 60, having sensitive skin (dry, couperose, problematic, prone to irritation) were selected for dermatological tests. All test subjects selected for the study met the requirements for admission to the study and signed a consent to participate voluntary in the study, and were informed about the purpose of the study, how it was conducted and about possible side effects. During the examination, the test subjects were under the constant care of a dermatologist.

The readings of the patch tests were made according to the International Contact Dermatitis Research Group (ICDRG).

**Table 8 - Graphical reading scale of the patch test according to ICDRG**

| | |
|---|---|
| 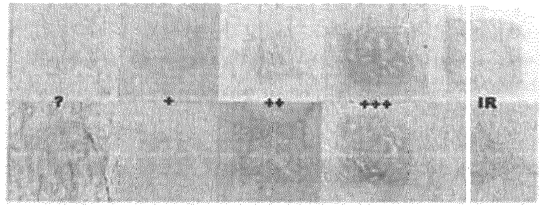 | |
| **(-)** negative | no reaction |
| **(?)** doubtful reaction | subtle erythema, palpable undetectable erythema spot |
| **(+)** weak reaction | palpable erythema, suggesting moderate swelling/infiltration, there may be papules, no blisters |
| **(++)** severe reaction | severe swelling, infiltration, papules, blisters |
| **(+++)** very severe reaction | blisters, erosion, ulceration |
| **(IR)** irritation reaction | shiny skin, dry skin, eczema, erythema |

In the group of 15 test subjects who were tested, no contact allergy or irritation reactions were reported after using the product. The lack of positive reactions proves that the tested product, i.e. the formulation according to the invention, does not exhibit irritating and sensitizing effects in contact with the skin.

### Example 9 - Testing and evaluation of the safety of using of the formulation according to the invention

The formulation according to the invention, prepared in Example 2, but taking into account the extended concentration ranges of the% of the individual ingredients, was subjected to detailed analysis and safety evaluation (Cosmetic Product Safety Report - data not shown).

In order to evaluate the safety of using of the formulation according to the invention, the following parameters were considered: qualitative and quantitative composition of the product with restrictions on the substances used (the substances were used in accordance with the Regulation EC No. 1223/2009), the physicochemical properties of the substances, their purity and possible impurities, stability of the final product and microbiological data, toxicological data of ingredients and risk of exposure to these substances, test results of the final product, purity and stability of the packaging material, information on adverse effects and product labelling.

The conducted research allowed to state that the formulation according to the invention, when used in accordance with its intended use, i.e., among others, for the use and/or care of skin prone to inflammations of various etiologies, and according to the method of its use, *i.e*. in the morning and in the evening, by applying an adequate amount of the formulation on the skin of the face and body, previously cleansed with a gentle product, does not pose a threat to human health and meets the legal requirements of current national regulations and the Regulation of the European Parliament and of the Council (EC) No. 1223/2009 of November 30, 2009.

According to the Safety Report, the toxicological profiles of the ingredients do not raise any objections. The calculated safety margins vastly exceed the value of 100. Raw materials for which the safety margin has not been calculated are substances commonly, and for many years, used in the cosmetics and/or food industry, and the history of their use does not report any negative effect on human health. They have been included in the list of safe cosmetic raw materials and safe drug ingredients. In the case of multi-component raw materials, the safety of all ingredients was addressed. The amount of substance to which the human body is exposed, in mg per kg of body weight per day (SED), was calculated for each component. For all the raw materials used, the SED values do not exceed 2.393 mg/kg/day, assuming daily use and taking into account their ability to penetrate human skin. The product is stable under normal storage conditions.

The applied substances listed in Annex V of the Regulation 1223/2009 (EC) were used in accordance with the requirements of the above-mentioned annexes, taking into account later regulations.

The product does not contain dyes or fragrances. Due to the content of propolis, which is a potential allergen, and trace amounts of alcohol, it is recommended to test the cream on a small area of the skin to check for possible hypersensitivity.

In summary, it was found that the formulation according to the invention is safe for human health when used in an intended manner and under foreseeable conditions.

### Example 10 - Testing the effects and effectiveness of the formulation according to the invention

The aim of the study was to evaluate the effect and effectiveness of the formulation according to the invention.

The study was conducted on a group of 20 test subjects at home.

| | | | | |
|---|---|---|---|---|
| Total number of test subjects: 20 | | | | |
| Division of the group by sex: | Women | 100% | Men | 0% |
| Division of the group by age: | Age 18-30 | 25% | | |
| | Age 31-40 | 45% | | |
| | Age 41-50 | 20% | | |
| | Age 51-60 | 10% | | |
| | Age > 61 | 0% | | |
| Skin type | sensitive | 70% | | |
| | with a tendency to damage | | | |
| | and inflammatory conditions | 30% | | |

Among the 70% of the testing group declaring sensitive type of skin, there were also test subjects with both sensitive and atopic, combination, couperose, dry or problematic skin. Among the 30% of the testing group, there were also test subjects with skin prone to irritation and damage to the epidermis and inflammation of various etiologies.

The test sample of the formulation according to the invention, obtained in Example 2, was applied in the morning and in the evening by applying the adequate amount of the formulation to the skin of the face and body, previously cleansed with a gentle product, for a period of 2 weeks. The test subjects were informed about the formulation recommendations. Due to the content of propolis, which is a potential allergen, and trace amounts of alcohol, it was recommended to carry out an initial test of the cream on a small area of the skin to check for possible hypersensitivity.

In case of skin irritation, it was recommended to reduce the amount of cream applied or use it once a day until the irritation symptoms subsided. If necessary, it was recommended to stop using the cream for a few days. The test subjects were informed that the cream should not be used in case of hypersensitivity to propolis or other ingredients of the formulation and that the cream should not be used after the expiry date stated on the packaging. The test subjects were also warned that the cream enhances the blood supply to the skin, so immediately after application, there may be pink and a slight tingling sensation, which subsides after a dozen, or so, minutes.

After 2 weeks of use, test subjects were asked to fill in (anonymously) an evaluation sheet about the formulation of the invention, and the results were summarized in Table 8 below.

**Table 9 - Results of the studies on the effect and effectiveness of the formulation according to the invention**

| **Tested feature/Effect** | **Example of answer** | **Number of test subjects** |
|---|---|---|
| Consistency | adequate | 95% |
| | too thick | 5% |
| | too thin | 0% |
| Spreadability | very easy | 15% |
| | easy | 70% |
| | moderate | 15% |
| | inconvenient | 0% |
| | very inconvenient | 0% |
| Efficiency | very high | 15% |
| | high | 60% |
| | moderate | 25% |
| | low | 0% |
| | very low | 0% |
| Absorption rate | very good | 10% |
| | good | 80% |
| | moderate | 10% |
| | poor | 0% |
| | very poor | 0% |
| Odour | very pleasant | 5% |
| | pleasant | 50% |
| | optimal | 40% |
| | unpleasant | 5% |
| | very unpleasant | 0% |
| Odour intensity | adequate | 95% |
| | too intense | 5% |
| | too faint | 0% |
| Caring effect | very noticeable | 10% |
| | noticeable | 65% |
| | moderate | 25% |
| | inconsiderable | 0% |
| | imperceptible | 0% |
| Moisturizing effect | very noticeable | 5% |
| | noticeable | 70% |
| | moderate | 25% |
| | inconsiderable | 0% |
| | imperceptible | 0% |
| Skin nourishing | very noticeable | 5% |
| | noticeable | 65% |
| | moderate | 30% |
| | inconsiderable | 0% |
| | imperceptible | 0% |
| Epidermis regeneration supporting | very noticeable | 5% |
| | noticeable | 70% |
| | moderate | 25% |
| | inconsiderable | 0% |
| | imperceptible | 0% |
| Epidermis softening | very noticeable | 5% |
| | noticeable | 75% |
| | moderate | 20% |
| | inconsiderable | 0% |
| | imperceptible | 0% |
| Feeling of comfort after using the product | very noticeable | 5% |
| | noticeable | 75% |
| | moderate | 20% |
| | inconsiderable | 0% |
| | imperceptible | 0% |
| Strengthening effect | very noticeable | 10% |
| | noticeable | 65% |
| | moderate | 25% |
| | inconsiderable | 0% |
| | imperceptible | 0% |
| Feeling of skin soothing | very noticeable | 10% |
| | noticeable | 65% |
| | moderate | 25% |
| | inconsiderable | 0% |
| | imperceptible | 0% |
| Improving skin hydration | very noticeable | 10% |
| | noticeable | 70% |
| | moderate | 20% |
| | inconsiderable | 0% |
| | imperceptible | 0% |
| Eliminating the feeling of the skin roughness and tightness | very noticeable | 20% |
| | noticeable | 60% |
| | moderate | 20% |
| | inconsiderable | 0% |
| | imperceptible | 0% |
| Improving skin elasticity | very noticeable | 20% |
| | noticeable | 60% |
| | moderate | 20% |
| | inconsiderable | 0% |
| | imperceptible | 0% |
| The ability of the product to restore a healthy appearance to the skin | very high | 15% |
| | high | 60% |
| | moderate | 25% |
| | low | 0% |
| | imperceptible | 0% |
| Improving the overall condition of the skin | very noticeable | 10% |
| | noticeable | 65% |
| | moderate | 25% |
| | inconsiderable | 0% |
| | imperceptible | 0% |

**Table 10 - Results of the study of adverse effects when using the formulation according to the invention**

| **Tested feature/Effect** | **Number of test subjects [%]** | |
|---|---|---|
| | **NO** | **YES** |
| Are there any side effects after using the product? Irritation, redness, itching, etc.? | 100 | 0 |

The specific parameters of the formulation according to the invention were positively evaluated by the test subjects. The product has an adequate consistency (95% of responses), high and very high efficiency (75% of responses), it is easy and very easy to be spread (85% of responses) and absorbed (90% of responses). Odour intensity was assessed as adequate (95% of responses). The formulation also exhibits a noticeable and very noticeable moisturizing effect, softening effect and the epidermis supporting effect (75%, 80%, 75% of responses, respectively). Most of the respondents confirmed that the product gives a noticeable and very noticeable feeling of comfort and soothing to the skin (80% and 75% of responses). In addition, the formulation according to the invention noticeably and very noticeably reduces the feeling of roughness and tightness of the skin (80% of the response), and also exhibits a high and very high ability to restore a healthy appearance to the skin (75% of the responses). The overall condition of the skin improved noticeably and very noticeably in 75% of the test subjects, and 100% of them did not notice any adverse reactions after the formulation use.

The above-cited data confirm that the use of a high concentration of omega-3, omega-6 and omega-9 polyunsaturated fatty acid ethyl esters of plant origin in the formulation according to the invention resulted in an unexpectedly high formulation effectiveness.

Ethyl esters of omega-3, omega-6 and omega-9 polyunsaturated fatty acids of plant origin, in combination with three vegetable oils: camelina, evening primrose and calendula oils, additionally support the process of the epidermis regeneration and enhance anti-inflammatory effects. The combination of these ingredients, in the adequate proportions, allowed to obtain a formulation of unexpectedly high effectiveness, which provides an exceptionally high anti-inflammatory and regenerative effect.

The unique composition of linseed oil esters in combination with three vegetable oils: evening primrose, camelina and calendula oils surprisingly effectively supports the process of the epidermis regeneration. Evening primrose oil, calendula oil and α-linolenic acid have anti-inflammatory and antibacterial properties, which is particularly important in the care of skin prone to inflammations. The omega 3,6,9 esters contained in the formulation are necessary to restore the proper hydro-lipid coat of the skin. Esters of omega 3 and 6 acids (esters of α-linolenic acid and linoleic acid) improve skin hydration, eliminate the feeling of the skin roughness and tightness, and reduce the predisposition to adverse changes of the epidermis. Omega 3 and 6 esters are absorbed by the stratum corneum and build into the intercellular cement, reducing water loss through the epidermis. They increase skin elasticity and restore its healthy appearance. Omega 9 esters (oleic acid esters) have moisturizing and softening properties.

Additionally, the formulation according to the invention does not induce contact allergy or any irritant reactions or other side effects such as redness, or itching.

Unexpectedly, it turned out that replacing ingredients of animal origin with ingredients of plant origin brings a surprising result in the form of a very noticeable improvement of the skin condition, resulting from the unexpected reduction of inflammation and improvement of many skin parameters, as well as an instant restoration of skin balance, skin nourishment and strength to make it more resistant to damage.

The above data indicate that the formulation according to the invention, due to its effectiveness and safety of use, can successfully replace formulations containing ingredients of animal origin, such as esters of DHA and EPA of animal origin. Thus, the technical problem of the invention has been successfully solved.

The above data show that the formulation according to the invention containing ethyl esters of plant-derived omega-3, 6 and 9 polyunsaturated acids is exceptionally effective in the care of skin prone to inflammations of various etiologies, in alleviating skin inflammation, in combating skin problems related to inflammation and in treating skin problems related to inflammation and enhancing skin regeneration.

The formulation according to the invention is therefore suitable for alleviating skin inflammations of various etiologies, for example atopic dermatitis, seborrheic dermatitis, contact dermatitis, folliculitis, acne, psoriasis, skin irritation or other skin conditions.

### Example 11 - Testing the effects and effectiveness of the formulation according to the invention

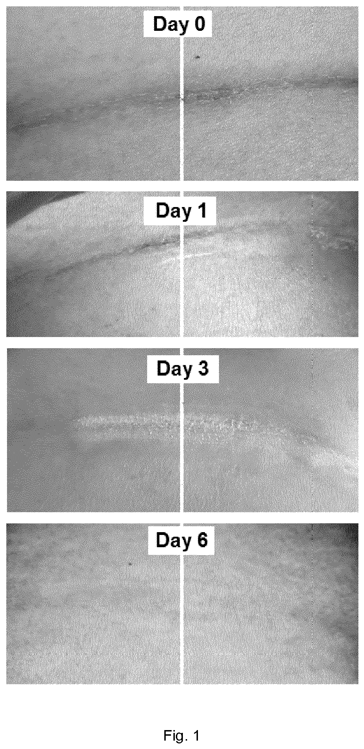

An additional test was carried out to evaluate the effects and effectiveness of the formulation of the invention. The study was conducted on a group of 8 patients of different ages, with each patient in the test group struggling with skin inflammations of various etiologies, as described below. The study was conducted at home. The formulation was applied a minimum of twice daily or more frequently as needed. The effectiveness of the formulation according to the invention was tested and documented below in Examples 11-1 to 11-8 and also in Figures 1-8, respectively.

**Example 11-1.** In an elderly, bedridden person spontaneously cracking skin on the abdomen was a recurring problem that could not be treated. The lesions lasted for many weeks despite the previously used therapies. The formulation according to the invention was applied on the lesion 3-4 times a day with for about 9 days. Photos were taken at three-day intervals. After 10 days, the skin was completely healed, and there were basically no sign of the lesion.

Fig. 1 shows the effect of the formulation according to the invention on cracking non-healing abdominal skin of an elderly and bedridden person.

**Example 11-2.** In a 5-year-old girl with a very strong food and inhalation allergy, manifested by severe asthma and skin lesions, the formulation according to the invention, which was applied on lichen that appeared as a result of drugs administration, completely eliminated the lesions after two days. Already, after the first application, a significant difference could be noticed. At first, the lesion seemed to have flared up, and after an hour it began to fade. On the second day, the lesion was already very faded, but the next day it was completely gone.

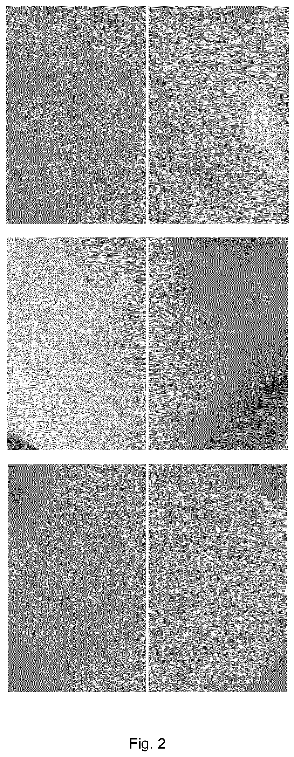

Fig. 2 shows the effect of the formulation according to the invention on lichen and cheek skin lesions associated with severe food and inhalation allergies in a 5-year-old girl.

**Example 11-3.** In a 9-year-old boy, for no apparent reason, lesions appeared on the ear that manifested itself by stinging sensation, burning, peeling of the epidermis and cracking of the skin. The lesion was treated with the formulation according to the invention at bedtime and again the next day. After a few hours, the skin returned to normal state.

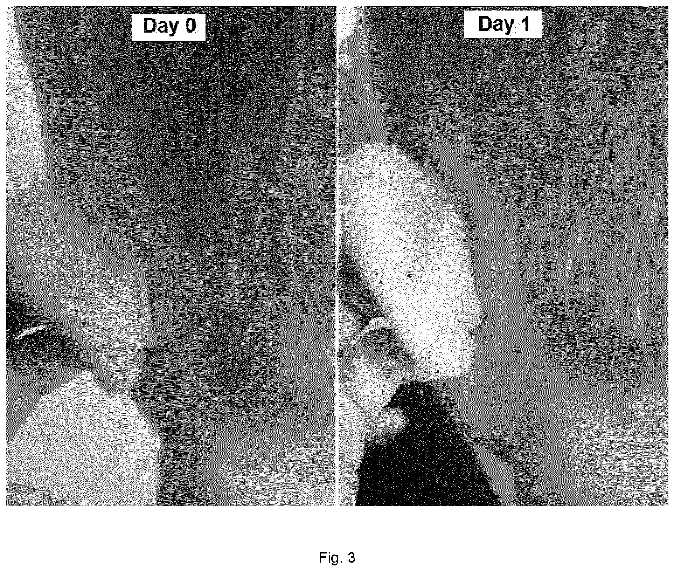

Fig. 3 shows the effect of the formulation according to the invention on atopic skin lesions of the ear, i.e. inflammation and scaly skin in a 9-year-old boy.

**Example 11-4.** In a middle-aged women, skin problems on the hands have persisted for years. Ulcerative and cracking lesions appear regularly every dozen of weeks, and the overall destruction of the skin on the hands has even led to a partial disappearance of fingerprints. After using the formulation according to the invention, the visible ulcerative lesion disappeared after a week, and after applying the cream on the whole hands for the next three weeks, not only all the lesions regressed, but no new ones appeared. Much of the fingerprints reappeared on the fingers.

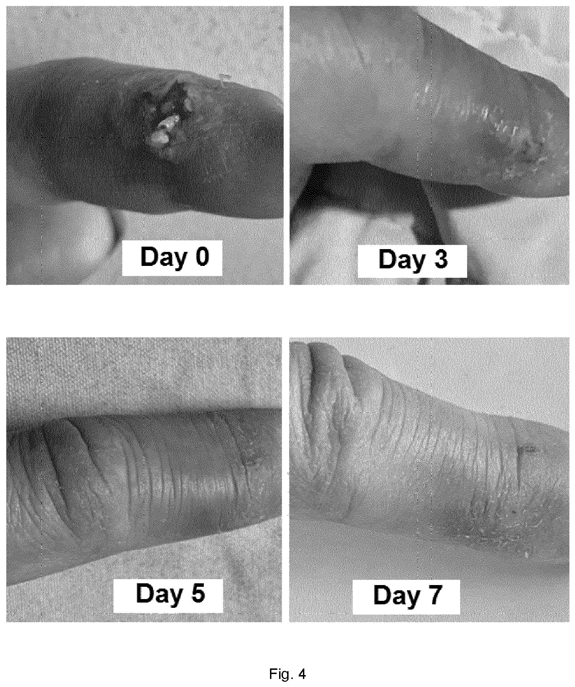

Fig. 4 shows the effect of the formulation according to the invention on cracking and ulcerated skin on the fingers of a middle-aged woman.

**Example 11-5.** In a middle-aged woman suffering from AD since childhood, the symptoms took the form of extremely dry skin, which caused cracking and bleeding, among others, on the calves. Additionally, there were painful, itchy and bleeding eczema on the chest and abdomen. After using the formulation according to the invention, the itching subsided almost immediately. The skin seemed less inflamed or reddened. After another few hours, the eczema stopped bleeding. The very next day they dried up, began to absorb and turned pale. After three days, the skin regained its natural colour, and there was no sign of the lesions.

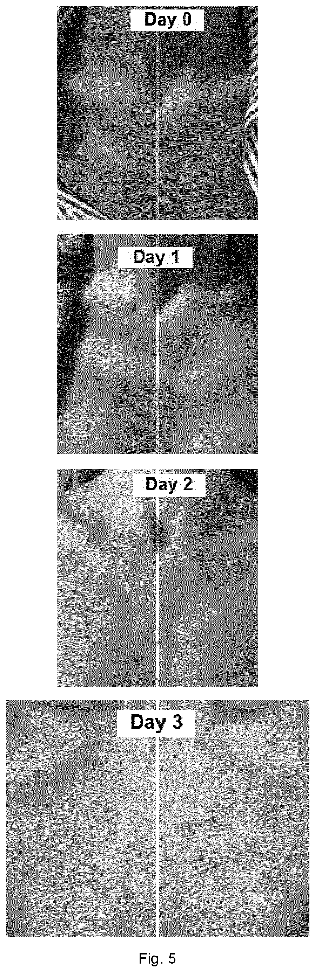

Fig. 5 shows the effect of the formulation according to the invention on atopic dermatitis manifested by painful, bleeding and itchy eczema in a middle-aged woman.

**Example 11-6.** In a 37-year-old man, the symptoms of bacterial folliculitis intensified from the age of twenty. The condition developed to such an extent that painful pustules covered almost the entire head, which even made it difficult to sleep. Treatment with antibiotics and steroids brought relief after a few days and for a few days. After stopping the antibiotics, the condition recurred. After applying the formulation according to the invention to the scalp, the itching disappeared immediately. After three hours, the redness of the head in general faded significantly. On the third day, mainly scabs and a few red discolorations remained on the head, and on the fifth day only scars remained in the areas of ulcers and pustules, which became more pale and faded each day. With the prophylactic use of the formulation twice a week, the skin remains under control and skin problems do not recur.

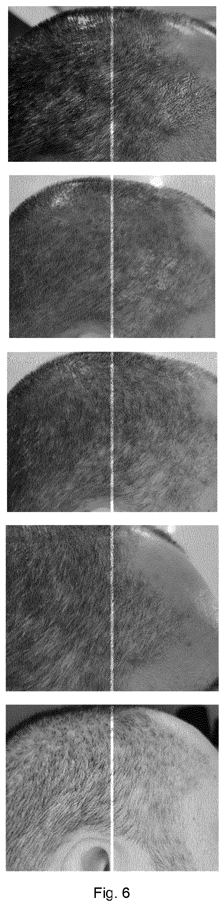

Fig. 6 shows the effect of the formulation according to the invention on acute folliculitis in a 37-year-old man.

**Example 11-7.** In the infant, increasing lesions in the area of the eyebrows at some point took the form of scabs moistened with plasma exudate. The formulation according to the invention was applied on the lesion once. A moment after the application, the affected skin reddened more strongly, then turned pale after 15 minutes. The skin lesion almost completely disappeared 55 minutes after application of the formulation

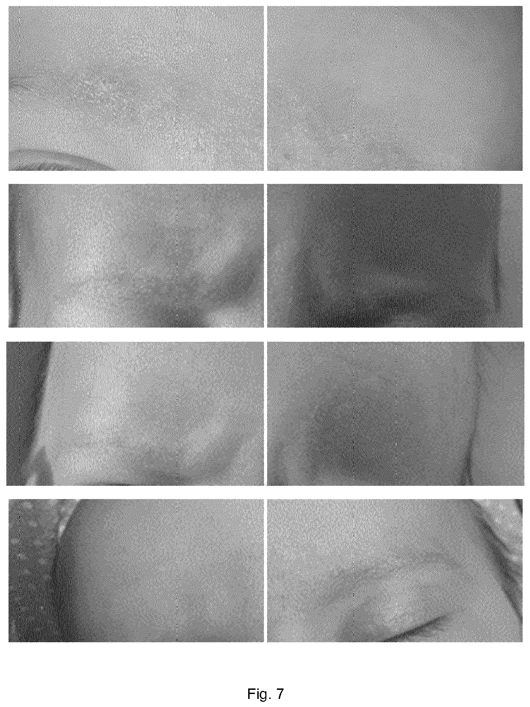

Fig. 7 shows the effect of the formulation according to the invention on skin inflammation associated with food allergy in an infant.

**Example 11-8.** In a one-and-a-half-year-old boy who had been treated with various ointments, including steroids, which had not worked, the formulation according to the invention first caused reddening of the lesions, and after some time the symptoms subsided, the lesions got dry and peeled off. With the prophylactic use of the formulation every other day, the lesions did not recur.

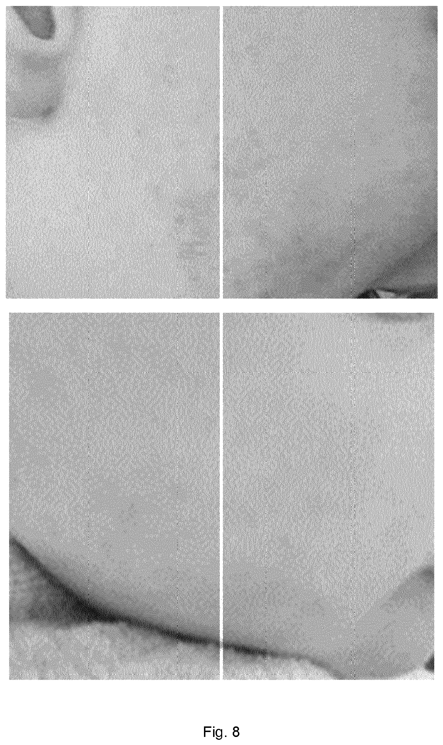

Fig. 8 shows the effect of the formulation according to the invention on atopic dermatitis in a one and a half year old boy

## Claims

1. A formulation for external application containing alkyl esters of polyunsaturated fatty acids **characterized in that** it contains ethyl esters of omega-3, omega-6 and omega-9 polyunsaturated fatty acids exclusively of plant origin, in a concentration from 8% to 45% with respect to the total weight of the formulation.

2. A formulation according to claim 1, **characterized in that** the source of ethyl esters of omega-3, omega-6 and omega-9 polyunsaturated fatty acids is vegetable oil selected from the group comprising: linseed oil *(Linum* L.), borage oil (*Borago* L.), black cumin oil (*Nigella* L.), pumpkin seed oil (*Cucurbita* L.), walnut oil (*Juglans L.)* and chia seed oil *(Salvia L.).*

3. A formulation according to claim 2, **characterized in that** the source of ethyl esters of omega-3, omega-6 and omega-9 polyunsaturated fatty acids is linseed oil (*Linum* L.)*.*

4. A formulation according to claim 3, **characterized in that** it contains ethyl ester of α-linolenic acid (omega-3), ethyl ester of linoleic acid (omega-6), ethyl ester of oleic acid (omega-9), ethyl ester of palmitic acid and ethyl ester of stearic acid, in the ratio 57% : 16% : 15% : 7% : 5%, respectively.

5. A formulation according to any one of the preceding claims 1-4, **characterized in that** it additionally contains at least one cosmetic vegetable oil.

6. A formulation according to claim 5, **characterized in that** it contains:
a) ethyl esters of omega-3, omega-6 and omega-9 polyunsaturated fatty acids derived from linseed oil (*Linum* L.), in a concentration of 8% to 45 wt.%; and
b) camelina oil (*Camelina* L.), in a concentration of 0.05% to 5 wt.%; and/or
c) evening primrose oil (*Oenothera* L.), in a concentration of 0.05% to 3 wt.%; and/or
d) calendula oil extract (*Calendula* L.), in a concentration of 0.01% to 4 wt.%,
wherein wt.% defines the concentration of a given ingredient with respect to the total weight of the formulation.

7. A formulation according to any one of the claims 1-6, **characterized in that** it further contains at least one additional active ingredient selected from the group comprising: D-panthenol, propolis, collagen, aloe extract, ozone, and copper.

8. A formulation according to any of the claims 1-7, **characterized in that** it is a cosmetic formulation or a pharmaceutical formulation.

9. A formulation according to any one of the claims 1-8, **characterized in that** it further contains cosmetically or pharmaceutically acceptable additives, such as moisturizers, stabilizers, preservatives, emulsifiers, emollients, antioxidants, fragrances, or solvents.

10. A formulation according to any of the claims 1-9, **characterized in that** it is in the form of a cream, oil, gel, or emulsion.

11. A formulation according to claim 10, **characterized in that** it is in the form of a water-in-oil emulsion.

12. A formulation as defined in claims 1-11 for use for care and/or supporting treatment and/or treatment of skin prone to inflammations of various etiologies, including alleviating skin inflammation and/or combating skin problems associated with inflammation and/or enhancing skin regeneration.

13. A formulation for use according to claim 12, wherein the skin prone to inflammations of various etiologies includes atopic skin, sensitive skin, combination skin, couperose skin, dry skin, problematic skin, skin prone to irritation and the epidermis damage.

14. A formulation for use according to claims 12-13, wherein the inflammation of the skin includes atopic dermatitis, seborrheic dermatitis, contact dermatitis, folliculitis, acne, psoriasis, skin irritation, and skin problems.

15. Use of ethyl esters of omega-3, omega-6 and omega-9 polyunsaturated fatty acids of plant origin in a preparation of a formulation for external use for care and/or supporting treatment and/or treatment of skin prone to inflammations of various etiologies, including alleviating skin inflammation and/or combating skin problems associated with inflammation and/or enhancing skin regeneration, wherein a concentration of ethyl esters of omega-3, omega-6 and omega-9 polyunsaturated fatty acids of plant origin in a formulation for external use ranges from 8 to 45% with respect to the total weight of the formulation.

## Patentansprüche

1. Formulierung zur äußeren Anwendung, die Alkylester von mehrfach ungesättigten Fettsäuren enthält, **dadurch gekennzeichnet, dass** sie Ethylester von mehrfach ungesättigten Omega-3-, Omega-6- und Omega-9-Fettsäuren ausschließlich pflanzlichen Ursprungs in einer Konzentration von 8% bis 45%, bezogen auf das Gesamtgewicht der Formulierung, enthält.

2. Formulierung nach Anspruch 1, **dadurch gekennzeichnet, dass** die Quelle für Ethylester von mehrfach ungesättigten Omega-3-, Omega-6- und Omega-9-Fettsäuren Pflanzenöl ist, das aus der Gruppe ausgewählt ist, die Folgende umfasst: Leinöl (*Linum L*.)*,* Borretschöl (*Borago* L.), Schwarzkümmelöl (*Nigella L*.)*,* Kürbiskernöl (*Cucurbita L*.)*,* Walnussöl (*Juglans L.*) und Chiasamenöl (*Salvia L.*)*.*

3. Formulierung nach Anspruch 2, **dadurch gekennzeichnet, dass** die Quelle für Ethylester von mehrfach ungesättigten Omega-3-, Omega-6- und Omega-9-Fettsäuren Leinöl (*Linum L.*) ist.

4. Formulierung nach Anspruch 3, **dadurch gekennzeichnet, dass** sie Ethylester der α-Linolensäure (Omega-3), Ethylester der Linolsäure (Omega-6), Ethylester der Ölsäure (Omega-9), Ethylester der Palmitinsäure und Ethylester der Stearinsäure im Verhältnis von 57% enthält: entsprechend 16%: 15%: 7%: 5%.

5. Formulierung nach einem der vorhergehenden Ansprüche 1-4, **dadurch gekennzeichnet, dass** sie zusätzlich mindestens ein kosmetisches Pflanzenöl enthält.

6. Formulierung nach Anspruch 5, **dadurch gekennzeichnet, dass** sie Folgende beinhaltet:
a) Ethylester von mehrfach ungesättigten Omega-3-, Omega-6- und Omega-9-Fettsäuren aus Leinöl (*Linum L*.) in einer Konzentration von 8 bis 45 Gew.-%; sowie
b) Leindotteröl (*Camelina L*.) in einer Konzentration von 0,05 bis 5 Gew.-%; und/oder
c) Nachtkerzenöl (*Oenothera L.*) in einer Konzentration von 0,05 bis 3 Gew.-%; und/oder
d) Ringelblumenölextrakt (*Calendula L.*) in einer Konzentration von 0,01 bis 4 Gew.-%, wobei Gew.-% die Konzentration eines bestimmten Inhaltsstoffs in Bezug auf das Gesamtgewicht der Formulierung definiert.

7. Formulierung nach einem der Ansprüche 1-6, **dadurch gekennzeichnet, dass** sie ferner mindestens einen zusätzlichen Wirkstoff enthält, der aus der Gruppe ausgewählt ist, die Folgende umfasst: D-Panthenol, Propolis, Kollagen, Aloe-Extrakt, Ozon und Kupfer.

8. Formulierung nach einem der Ansprüche 1-7, **dadurch gekennzeichnet, dass** es sich um eine kosmetische Formulierung oder eine pharmazeutische Formulierung handelt.

9. Formulierung nach einem der Ansprüche 1-8, **dadurch gekennzeichnet, dass** sie zusätzlich kosmetisch oder pharmazeutisch verträgliche Zusatzstoffe wie Feuchtigkeitsspender, Stabilisatoren, Konservierungsmittel, Emulgatoren, Erweichungsmittel, Antioxidantien, Duftstoffe oder Lösungsmittel enthält.

10. Formulierung nach einem der Ansprüche 1-9, **dadurch gekennzeichnet, dass** sie in Form einer Creme, eines Öls, eines Gels oder einer Emulsion vorliegt.

11. Formulierung nach Anspruch 10, **dadurch gekennzeichnet, dass** sie in Form einer Wasser-in-Öl-Emulsion vorliegt.

12. Formulierung nach den Ansprüchen 1-11 zur Verwendung zur Pflege und/oder Unterstützung der Behandlung und/oder Behandlung von Haut, die für Entzündungen verschiedener Ätiologien anfällig ist, einschließlich der Linderung von Hautentzündungen und/oder zur Bekämpfung von Hautproblemen, die mit Entzündungen verbunden sind, und/oder zur Verbesserung der Hautregeneration.

13. Formulierung zur Verwendung nach Anspruch 12, wobei die Haut, die für Entzündungen verschiedener Ätiologien anfällig ist, atopische Haut, empfindliche Haut, Mischhaut, Couperose-Haut, trockene Haut, problematische Haut, Haut, die für Reizungen anfällig ist, und Epidermisschädigungen umfasst.

14. Formulierung zur Verwendung nach den Ansprüchen 12-13, wobei die Hautentzündung atopische Dermatitis, seborrhoische Dermatitis, Kontaktdermatitis, Follikulitis, Akne, Psoriasis, Hautreizung und Hautprobleme umfasst.

15. Verwendung von Ethylestern von mehrfach ungesättigten Omega-3-, Omega-6- und Omega-9-Fettsäuren pflanzlichen Ursprungs in einer Zubereitung einer Formulierung zur äußeren Anwendung zur Pflege und/oder unterstützenden Behandlung und/oder Behandlung von Haut, die für Entzündungen verschiedener Ätiologien anfällig ist, einschließlich der Linderung von Hautentzündungen und/oder der Bekämpfung von Hautproblemen verbunden mit Entzündungen und/oder der Verbesserung der Hautregeneration, wobei eine Konzentration von Ethylestern von mehrfach ungesättigten Omega-3-, Omega-6- und Omega-9-Fettsäuren pflanzlichen Ursprungs in einer Formulierung zur äußeren Anwendung im Bereich von 8 bis 45%, bezogen auf das Gesamtgewicht der Formulierung, liegt.

## Revendications

1. Une formulation pour application externe contenant des esters alkyliques d'acides gras polyinsaturés, **caractérisée en ce qu'elle** contient des esters éthyliques d'acides gras polyinsaturés oméga-3, oméga-6 et oméga-9 exclusivement d'origine végétale, à une concentration comprise entre 8 % et 45 % par rapport au poids total de la formulation.

2. Formulation selon la revendication 1, **caractérisée en ce que** la source d'esters éthyliques d'acides gras polyinsaturés oméga-3, oméga-6 et oméga-9 est une huile végétale choisie dans le groupe comprenant : l'huile de lin (*Linum L.*)*,* l'huile de bourrache (*Borago L.), l*'huile de nigelle (*Nigella L.*)*,* l'huile de pépins de courge (*Cucurbita L*.)*,* l'huile de noix (*Juglans L.*) et l'huile de chia (*Salvia L.*)*.*

3. Formulation selon la revendication 2, **caractérisée en ce que** la source d'esters éthyliques d'acides gras polyinsaturés oméga-3, oméga-6 et oméga-9 est l'huile de lin (*Linum L.).*

4. Formulation selon la revendication 3, **caractérisée en ce qu'elle** contient de l'ester éthylique d'acide α-linolénique (oméga-3), de l'ester éthylique d'acide linoléique (oméga-6), de l'ester éthylique d'acide oléique (oméga-9), de l'ester éthylique d'acide palmitique et de l'ester éthylique d'acide stéarique, dans un rapport de 57 % : 16 % : 15 % : 7 % : 5 %, respectivement.

5. Formulation selon l'une quelconque des revendications 1 à 4 précédentes, **caractérisée en ce qu'elle** contient en outre au moins une huile végétale cosmétique.

6. Formulation selon la revendication 5, **caractérisée en ce qu'elle** contient :
a) des esters éthyliques d'acides gras polyinsaturés oméga-3, oméga-6 et oméga-9 dérivés de l'huile de lin (*Linum L*.*)*, à une concentration comprise entre 8 % et 45 % en poids ; et
b) de l'huile de caméline (*Camelina L.),* à une concentration comprise entre 0,05 % et 5 % en poids
; et/ou
c) de l'huile d'onagre (*Oenothera L.),* à une concentration comprise entre 0,05 % et 3 % en poids
; et/ou
d) de l'extrait d'huile de calendula (*Calendula L.),* à une concentration comprise entre 0,01 % et 4 % en poids,
où le pourcentage en poids définit la concentration d'un ingrédient donné par rapport au poids total de la formulation.

7. Formulation selon l'une quelconque des revendications 1 à 6, **caractérisée en ce qu'elle** contient en outre au moins un ingrédient actif supplémentaire choisi dans le groupe comprenant : le D-panthénol, la propolis, le collagène, l'extrait d'aloès, l'ozone et le cuivre.

8. Formulation selon l'une quelconque des revendications 1 à 7, **caractérisée en ce qu'il** s'agit d'une formulation cosmétique ou d'une formulation pharmaceutique.

9. Formulation selon l'une quelconque des revendications 1 à 8, **caractérisée en ce qu'elle** contient en outre des additifs cosmétiquement ou pharmaceutiquement acceptables, tels que des agents hydratants, des stabilisants, des conservateurs, des émulsifiants, des émollients, des antioxydants, des parfums ou des solvants.

10. Formulation selon l'une quelconque des revendications 1 à 9, **caractérisée en ce qu'elle** se présente sous la forme d'une crème, d'une huile, d'un gel ou d'une émulsion.

11. Formulation selon la revendication 10, **caractérisée en ce qu'elle** se présente sous la forme d'une émulsion eau dans huile.

12. Formulation telle que définie dans les revendications 1 à 11, destinée à être utilisée pour le soin et/ou le traitement d'appoint et/ou le traitement des peaux sujettes à des inflammations de diverses étiologies, y compris pour soulager l'inflammation cutanée et/ou lutter contre les problèmes cutanés associés à l'inflammation et/ou améliorer la régénération cutanée.

13. Formulation destinée à être utilisée conformément à la revendication 12, dans laquelle la peau sujette à des inflammations de diverses étiologies comprend la peau atopique, la peau sensible, la peau mixte, la peau couperosée, la peau sèche, la peau à problèmes, la peau sujette à l'irritation et les lésions épidermiques.

14. Formulation destinée à être utilisée conformément aux revendications 12 à 13, dans laquelle l'inflammation de la peau comprend la dermatite atopique, la dermatite séborrhéique, la dermatite de contact, la folliculite, l'acné, le psoriasis, l'irritation cutanée et les problèmes cutanés.

15. Utilisation d'esters éthyliques d'acides gras polyinsaturés oméga-3, oméga-6 et oméga-9 d'origine végétale dans une préparation d'une formulation à usage externe pour le soin et/ou le traitement d'appoint et/ou le traitement de la peau sujette à des inflammations de diverses étiologies, y compris l'atténuation de l'inflammation cutanée et/ou la lutte contre les problèmes cutanés associés à l'inflammation et/ou l'amélioration de la régénération cutanée, dans laquelle une concentration d'esters éthyliques d'acides gras polyinsaturés oméga-3, oméga-6 et oméga-9 d'origine végétale dans une formulation à usage externe varie de 8 à 45 % par rapport au poids total de la formulation.
